# EUROPEAN PATENT APPLICATION

(11) **EP 1 334 991 A1**
(43) Date of publication of application: **13.08.2003**
(21) Application number: 02075566.6
(22) Date of filing: 08.02.2002
(51) Int. Cl.: C08G 77/04, C08G 77/50, A61L 27/18, G02B 1/04

(54) **High refractive index flexible silicone**

(71) Applicant: Ophtec B.V., 9728 NR Groningen (NL)
(72) Inventor: Verbruggen, Miriam Adrienne Lambertina, 8014 WJ Zwolle (NL); Flipsen, Theodorus Adrianus Cornelius, 9723 DR Groningen (NL); van der Flier, Eelco Christoffer Brian, 7512 GV Enschede (NL); Smit, Hendrik, 9617 EM Harkstede (NL)
(74) Representative: Prins, Adrianus Willem

(57) **Abstract**

The present invention discloses to a high refractive index polysiloxane homopolymer comprising refractive index modifying groups chemically bonded in clustered configuration to the polysiloxane backbone. The invention also discloses methods for the preparation of such high refractive index polysiloxane homo-polymers as well as co-polymers prepared thereof. A polysiloxane according to the invention is very suitable for use as a material for the production of intra-ocular lenses.

## Description

### Field of the invention

The invention relates to a silicone material with a high refractive index. More particularly, it relates to a flexible silicone material with a high refractive index for use as an intra-ocular lens material.

### Background of the invention

Prosthetic implant lenses are widely used to replace natural lenses that are affected by cataract. Cataract is the leading cause of blindness in the industrialized and developing world. The standard medical procedure for treatment is to remove the natural cloudy lens by elective cataract surgery and replace it with an intra-ocular lens.

Currently, flexible silicone is progressively replacing the classical polymethyl-methacrylate (PMMA) material and its derivative polymers as an intra-ocular lens implant material. Continued perfection of the cataract operation has brought new techniques that remove the natural lens through a self-sealing incision so tiny that no sutures are required. Thin, flexibly foldable implant materials permit the insertion of the implant lens through the same small size self-sealing incision of the cataract operation, which has the important advantage to considerably reduce the risk of post-operative complications.

Except for a preferred requirement of flexibility, materials for intra-ocular lenses should be clear, highly transparent and should allow for the manufacturing of lenses with high refractive indices. An intra-ocular lens with a high refractive index has the advantage that it can be thinner at the same dioptric power as an intra-ocular lens with lower refractive index.

In order to increase the refractive index of intra-ocular lenses copolymers of polysiloxane and polymethacrylates may be prepared. Also, sulfur compounds can be introduced into the siloxane polymer. But despite these modifications, the refractive index of silicone materials is still limited, and a thin lens thereof does not provide much optical power.

It is recognized that the inclusion of diphenyl siloxane or phenyl-methyl siloxane into a polysiloxane results in a polymer of higher refractive index as disclosed in US 3,996,189. Introduction of aromatic groups is now a general approach to increase the refractive index of intra-ocular lens materials while maintaining biocompatibility. Although other refractive index modifying groups such as cyclo-alkyl groups or aromatic groups, optionally combined with phenyl groups (JP2000017176) or phenol groups (US 5,541,278), can also be used, conventional co-polymers for intra-ocular lenses consist of dimethylsiloxane-phenylmethylsiloxane co-polymers or dimethylsiloxane-diphenylsiloxane co-polymers as described in e.g. US 5,147,396, JP10305092, EP 0335312, WO 93/21245, WO 95/17460, US 5,444,106 and US 5,236,970.

By thus increasing the phenyl content of silicone co-polymers, materials with a relatively high refractive index can be obtained. At a phenyl content of approximately 15 mol %, the polydimethyl siloxane / methylphenyl siloxane co-polymer has a refractive index of 1.462 (Gu & Zhou, *Eur. Polymer J.* 34, pp. 1727-33 [1998]). However, such phenyl-containing siloxanes have the disadvantage of exhibiting reduced flexibility or elongation. The presence of phenyl groups attached to the alternating silicon-oxygen backbone of the siloxane causes the (co-)polymer to become relatively stiff and, despite their potential dioptric power, the suitability of such polymers as a material for intra-ocular lenses is greatly reduced.

In general, the introduction of aromatic groups, such as phenyl groups, in polysiloxane increases the glass transition temperature, or Tg, of the polymer, making it more hard and brittle and less flexible over a wider temperature range. This renders the material more vulnerable to cracking or breaking during folding and reduces the suitability of phenylated polysiloxanes as a material for intra-ocular lenses.

A well known remedy to this problem is to reinforce the lens and improve its mechanical properties by combining the polymer with a solid filler material. Mostly, finely powdered silica is used as a filler material for this purpose. This filler material has a refractive index of 1.46. Since differences in the refractive index of the filler material and the polymer are not allowable in an optical lens, the maximum refractive index of a lens containing such filler material is ultimately 1.46.

Therefore, the use of silicone as an intra-ocular lens implant material and particularly the development of silicone with the enhanced material characteristics that would support broadening of such use is determined, at large, by the maximum attainable refractive index of the material and its associated flexibility.

One method of reducing the glass transition temperature of phenyl-modified polysiloxanes is to link the phenyl-groups to the silicon-oxygen backbone by alkanediyl-bridges. Such modification of polysiloxanes is known from US 4,780,510 wherein a hydride/vinyl reaction pair is used. US 5,233,007, WO 93/21258 and US 5,420,213 describe a process wherein phenyl groups are introduced in tetramethyl cyclotetra siloxane via an addition reaction with styrene to produce a tetramethylstyryl cyclotetra siloxane monomer. Polymerization or co-polymerization with other siloxanes can result in a (co)polymer with a refractive index of about 1.54. However, the amount of phenyl groups or the number of phenyl-modifications that can be introduced is still limited and silicone-based intra-ocular lens implant materials with higher refractive indices are still needed.

Producing high refractive index polysiloxanes from monomers that contain the refractive index modifying groups is generally perceived as difficult, resulting in expensive or essentially less pure polymers. Additionally, the preparation of a siloxane (co-)polymer with a predetermined high value for the refractive index is perceived as very difficult to control.

Another problem with the production of intra-ocular lens material with a high refractive index is associated with the molecular weight of the final polymer and the attainable mechanical strength. In general, styrene-modified polysiloxanes exhibit very low viscosity compared to polysiloxane materials with a low refractive index. This low viscosity is probably due to a low molecular weight of the polymer, which in turn results in difficulties with achieving the levels of cross-linking required for high mechanical strength rubbers. It is known, for example, that diphenyl cyclosiloxanes cannot easily be polymerized to high molecular weight polymers as a result of the unfavorable thermodynamics of the reaction from rings to chains (Journal of Polymer Science Part A: Polymer Chemistry, v35(10), p 1973). Only the application of non-equilibrium reaction conditions will yield the desired product, but such conditions are less suitable for large-scale production.

As such, the introduction of refractive-index modifying groups decreases the flexibility of the eventual polymer and lowers its mechanical strength due to the formation of relatively low molecular weight polysiloxanes.

### Summary of the invention

The present invention provides for a new class of polysiloxanes as well as new silicon materials with high refractive index values. High refractive index polysiloxane homo-polymers according to the present invention may contain very high numbers of refractive index modifying groups without compromising the flexibility and other important material characteristics of a silicone prepared thereof. By co-polymerizing the high refractive index polysiloxane homo-polymers of the present invention with other, conventional polysiloxanes, a high refractive index polysiloxane co-polymer may be obtained. This high refractive index polysiloxane co-polymer is a very suitable material for the manufacturing of intra-ocular lenses.

The present invention also provides methods for the preparation of high refractive index polysiloxane homo-polymers according to the invention, methods for preparing high refractive index polysiloxane co-polymers therewith and methods for the preparation of intra-ocular lenses.

The present invention provides high refractive index polysiloxane copolymers that can be prepared through a process that is relatively easy to control and that does not lead to large batch-to-batch quality variations. Such a process and the materials resulting thereof are required for the mass manufacturing of intra-ocular lenses.

Surprisingly it was found that the refractive index of siloxane polymers is increased considerably, when refractive index modifying groups are bonded to the siloxane backbone in a clustered configuration via an alkanediyl-bridge. Despite the presence of high numbers of refractive index modifying groups a high refractive index polysiloxane homo-polymer is obtained of which the mechanical properties are minimally affected. The advantageous material characteristics of silicone prepared therewith are essentially maintained.

The present invention relates to a high refractive index polysiloxane homo-polymer, comprising a clustered configuration of refractive index modifying groups chemically bonded to the polysiloxane backbone via an alkanediyl-bridge such that one alkanediyl-bridge binds at least two refractive index modifying groups to said backbone.

By introducing more than one refractive index modifying group at each alkanediyl-bridge, relatively high refractive indices can be attained whilst only a limited number of modifications is required. When, for example, a cluster of three phenyl groups is bonded to a polysiloxane backbone via an alkanediyl-bridge, the contribution to the refractive index per modification will be about three times as high as when a styrene is bonded to a polysiloxane backbone.

The attainable refractive indices of the new high refractive index polysiloxane homo-polymer material may reach such high values, that blending with conventional and even unmodified polysiloxanes is possible while maintaining a relatively high refractive index in the resulting co-polymer. Based on the high refractive index polysiloxane homo-polymers according to the invention therefore, high refractive index polysiloxane copolymers of high mechanical strength and with a high refractive index can be obtained. Such a co-polymer constitutes an excellent material for, e.g., intra-ocular lenses.

### Description of the drawings

Figure 1 represents a cyclic siloxane that may be used as a monomeric precursor for the preparation of a high refractive index polysiloxane homo-polymer according to the invention.
Figure 2 represents the clustered configuration wherein the refractive index modifying groups are positioned in the high refractive index polysiloxane homo-polymers or co-polymers of the present invention and identifies the polysiloxane backbone, the alkanediyl-bridge, the carrier component (X) and the refractive index modifying groups (Y).
Figure 3 represents an embodiments of the present invention wherein an addition reaction between triphenyl-silane and trivinyl pentamethyl cyclo tetrasiloxane and the resulting high refractive index polysiloxane homo-polymer is illustrated.

### Detailed description of the invention

As used herein, the term alkyl group refers to a straight-chain or a branched-chain alkyl radical containing from 1 to 20, preferably from 1 to 10, more preferable from 1 to 3, carbon atoms. The alkyl radical may be optionally substituted with one or more substituents that comprise of elements such as oxygen, nitrogen, sulfur, halogen (Cl, Br, I, F), hydrogen and phosphorus and may comprise alkoxy, hydroxy, amino, nitro or cyano.

The term (cyclo)alkyl group refers to an alkyl radical or a cyclic alkyl radical. The latter includes saturated or partially saturated monocyclic, bicyclic or tricyclic alkyl radicals wherein each cyclic moiety contains 3 to 8, preferably 4 to 8, carbon atoms. Examples of such radicals include methyl, ethyl, n-propyl, isopropyl, n-butyl, isobutyl, sec-butyl, tert-butyl, pentyl, isoamyl, hexyl, octyl, cyclopentyl, cyclopentenyl, cyclohexenyl, and cyclohexyl and are understood to include (cyclo)alkyl groups optionally substituted with substituents that comprise of elements such as oxygen, nitrogen, sulfur, halogen, hydrogen and phosphorous and may comprise alkyl, alkoxy, hydroxy, amino, nitro or cyano.

The term aromatic group refers to the monocyclic and polycyclic aromatic hydrocarbons, or arenes, and their substitution products such as benzene, naphthalene, toluene as well as to the hetero-aromatic, or aromatic heterocyclic structures, such as thiophene and pyridine.

The term aryl group refers to radicals comprising an aromatic or hetero-aromatic ring system derived from arenes by removal of a hydrogen atom from a ring carbon atom, such as a phenyl, naphtyl or anthracene radical which may optionally carry one or more substituents that comprise of elements such as oxygen, nitrogen, sulfur, halogen (Cl, Br, I, F), hydrogen and phosphorous and may comprise alkyl, alkoxy, hydroxy, amino, nitro or cyano. Examples of such radicals include phenyl, p-tolyl, 4-methoxyphenyl, 4-(tert-butoxy) phenyl, 4-chlorophenyl, 4-hydroxyphenyl, 1-naphtyl, and 2-naphtyl. It is to be noted that fused and connected rings, as well as 5, 6, 7 or 8 membered rings, such as cyclopentadienyl, imidazolyl, thiophenyl, thienyl, etc. are included. In the context of the present invention, aryl groups will be understood to include the arene-derived bivalent arylene groups, such as o-phenylene and the arynes, such as benzyne.

The term arylalkyl group means an alkyl radical as defined above in which one hydrogen atom is replaced by an aryl radical as defined above, such as benzyl, or 2-phenylethyl.

Allyl refers to propene radicals (CH₂)₂CH.

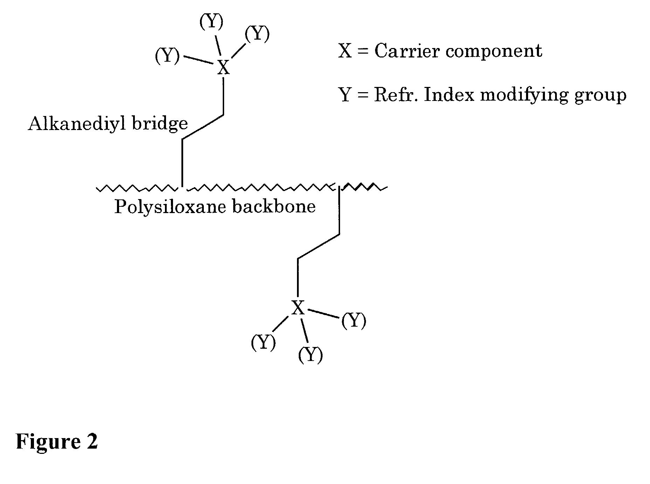

A high refractive index polysiloxane homo-polymer according to the invention may be prepared by chemically modifying a monomeric cyclic siloxane precursor through a vinyl/hydride addition reaction with a carrier component (X in figure 2) containing at least two refractive index modifying groups (Y in figure 2) in the presence of an addition reaction catalyst. Use may be made of monomeric cyclic siloxane precursors that contain reactive groups such as hydride groups or vinyl groups as a precursor material for a high refractive index polysiloxane homo-polymer. Such precursors are commercially available (e.g. Petrarch® product line, United Chemical Technologies, Inc., Bristol, Pennsylvania, USA) and support well controlled addition reactions according to the invention.

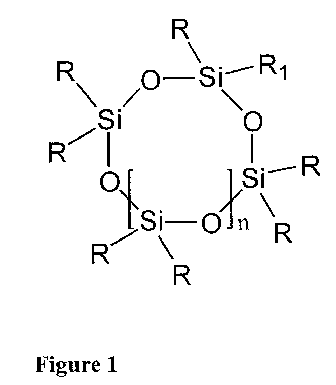

A monomeric cyclic siloxane precursor that is used in embodiments of the present invention may include three or more, preferably 3 to 6, more preferably 3 or 4 silicon atoms alternated with oxygen atoms in a cyclic structure such as presented in figure 1. In this figure, n is preferably 0, 1, 2 or 3, more preferably 0 or 1; R is independently alkyl, vinyl or hydride; R₁ is hydride or, in an alternative embodiment, vinyl. A monomeric cyclic siloxane precursor according to the invention may comprise from 1 to 12, preferable from 2 to 6, more preferable from 2 to 4 reactive groups in the form of vinyl or hydride groups.

A carrier component (X) can advantageously be bonded to a silicon atom of the precursor through a vinyl/hydride addition reaction, such as a hydrosilylation reaction. To support such a reaction, the precursor may contain one or more hydride groups bonded to silicon atoms of the cyclic siloxane, in which case the carrier component would comprise a reactive vinyl group. Upon ring-opening of the modified cyclic precursor, polymerization reactions between the monomers will result in a polysiloxane homo-polymer according to the present invention.

In an alternative embodiment, it is possible to use a precursor monomer, comprising one or more reactive vinyl groups bonded to the silicon atoms of the cyclic siloxane to which the carrier component with the refractive index modifying groups (Y) can effectively be bonded. In such a case, the carrier component would preferably comprise a vinyl reactive hydride group.

The position and distribution of the reactive groups in a precursor monomer is not critical.

One or more carrier components (X) containing at least two refractive index modifying groups (Y) can be bonded to each precursor monomer. In one embodiment a cyclic siloxane precursor is modified via an alkanediyl-bridge with only one carrier component (X) containing at least two refractive index modifying groups (Y) attached thereto.

In alternative embodiments several or all of the silicon atoms in a cyclic siloxane precursor molecule may have bonded two carrier components (X), each carrying at least two refractive index modifying groups (Y), via alkanediyl-bridges. This latter situation will result in high numbers of refractive index modifying groups in the eventual polysiloxane and a high refractive index of the silicone. The number of carrier components bonded to the precursor monomers may be selected such that a desired refractive index in the eventual polysiloxane material is obtained.

Based on the above, a large number of useful precursor materials for the preparation of high refractive index polysiloxane homo-polymers or copolymers according to the present invention can be identified by those skilled in the art. Among these are cyclic hydride-containing siloxane monomeric precursors. Also cyclic vinyl-containing siloxane precursors, such as pentavinyl pentamethyl cyclopenta siloxane, can be used in embodiments of the present invention. Particularly useful precursors are tetrahydro tetramethyl cyclosiloxane and tetravinyl tetramethyl cyclo tetrasiloxane.

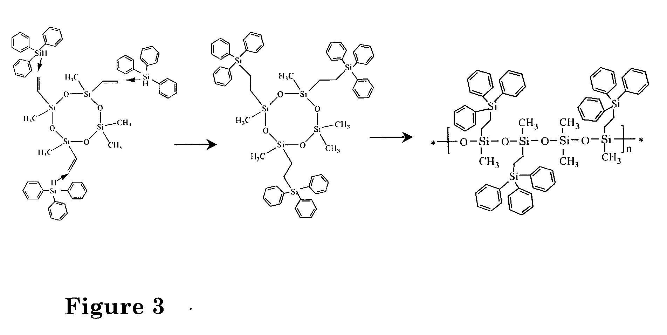

As stated, a carrier component containing at least two refractive index modifying groups is reacted with a monomeric cyclic siloxane precursor as described (figure 3).

Preferably, the bonding of the carrier component (X) containing at least two refractive index modifying groups (Y) results in a situation wherein it is separated or spaced apart from the silicon atom to which it is most directly bonded. Such spacing can very advantageously be accomplished by using an alkanediyl-bridge between the carrier component and the silicon atom. A very useful reaction pair is therefore formed by an hydride/vinyl reaction pair, whereby one member of said reaction pair is present on the monomeric cyclic siloxane precursor and the other member of said reaction pair is present on the carrier component that contains the refractive index modifying groups, because such a reaction results in an alkanediyl-bridge, in particular an ethanediyl-bridge.

An alternative reaction pair may comprise a hydride/allyl reaction pair, which also results in an alkanediyl-bridge, in particular a propanediyl-bridge.

In the light of the above, other suitable reaction pairs will be obvious to the person skilled in the art.

To obtain a high refractive index polysiloxane homo-polymer according to the invention, a large number of refractive index modifying groups (Y) can be used. Both cyclic, straight chain and branched chain hydrocarbons are suitable. Preferably, aromatic groups, aryl groups, arylalkyl groups, cycloalkyl groups and/or branched alkyl groups are used as refractive index modifying groups (Y) in embodiments of the present invention. More preferably, phenyl groups are used as refractive index modifying groups.

One carrier component (X) may carry two or more, preferably three, refractive index modifying groups (Y). The carrier component (X) may comprise a single or multi-atom structure. The carrier component (X) may suitably comprise a silicon atom to which the refractive index modifying groups (Y) are bonded, in which case the silicon atom serves as the central atom of a clustered configuration of refractive index modifying groups. However, other atoms may also be comprised by the carrier component (X). Instead of silicon, phosphorus, nitrogen, germanium or carbon may for example be used as the central atom of a clustered configuration of refractive index modifying groups. The choice for the carrier component (X) is not critical as long as at least two refractive index modifying groups (Y) can be bonded thereto and as long as it can be attached to a silicon atom of a cyclic siloxane via an alkanediyl-bridge.

Preferably, the carrier component is bonded to a cyclic siloxane monomer with the refractive index modifying groups already attached to the carrier component. A clustered configuration of refractive index modifying groups according to the invention therefore preferably comprises a carrier component (X) with the refractive index modifying groups (Y) already attached. A clustered configuration of refractive index modifying groups (X+Y) according to the invention can suitably be introduced into a cyclic siloxane monomer by using compounds such as triphenyl silane, diphenyl silane, triphenyl dimethyl disiloxane, diphenylalkyl dimethyl disiloxane, all of which will readily react with a vinyl-containing siloxane precursor and wherein carrier and refractive index modifying groups are combined.

Also very suitable compounds for introducing a clustered configuration of refractive index modifying groups into a cyclic siloxane monomer are compounds such as triphenylvinyl silane, diphenylalkylvinyl silane, phenyldialkylvinyl silane, triphenyl dimethylvinyl disiloxane, diphenyl ethene, 3,3,3-triphenyl-1-propene, 3,3-diphenyl-1-propene or any other vinyl reactive polyphenyls, all of which will readily react with a hydride-containing siloxane precursor.

Triphenyl silane and triphenyl vinyl silane are preferred compounds by which a clustered configuration of refractive index modifying groups can be introduced in polysiloxane precursors and/or polysiloxanes of the present invention. The person skilled in the art will readily understand from the above which alternative compounds may be used for introducing a clustered configuration of refractive index modifying groups into a cyclic siloxane monomer.

A preferred method for preparing a high refractive index polysiloxane homo-polymer of the invention comprises the steps of
a) chemically modifying a cyclic siloxane by performing an addition reaction in the presence of an addition reaction catalyst which reaction links to the siloxane backbone of said cyclic siloxane a clustered configuration of refractive index modifying groups via an alkanediyl-bridge such that at least two of said refractive index modifying groups are bound to said backbone via a single alkanediyl-bridge,
b) allowing ring opening of the cyclic siloxane and
c) facilitating subsequent polymerization of the resulting reaction products thereby obtaining the high refractive index polysiloxane homo-polymer.

The above described method for preparing a high refractive index polysiloxane homo-polymer harbors many advantages over methods of the prior art and can favorably be used for the preparation of high refractive index polysiloxane co-polymers.

Although it is possible to perform addition reactions to polysiloxanes, such additions are not envisioned in the context of the present invention. Addition reactions to polysiloxanes are generally difficult to control. In particular, the reaction efficiency, i.e. the degree to which the addition reaction has occurred, the fraction of the polymeric material modified, the amount and the distribution of reacted groups in the eventual polymer and therefore the refractive index as well as the quality of the eventual material is difficult to control.

Furthermore, in order to perform addition reactions to polymers, solvents are required to lower the viscosity of the preparation. The presence of the solvent, however, will also increase the risk of the introducing impurities into the preparation and increases the risk of associated negative, undesired side-reactions. As a further effect of the solvent and the associated dilution of reactive groups present in the reaction mixture, the reaction progresses at a lower rate and higher reaction temperatures are required to avoid long reaction periods.

When a clustered configuration of refractive index modifying groups, such as triphenylsilane, is reacted with a cyclic siloxane precursor, such as tetravinyl tetramethyl cyclo tetrasiloxane according to a method of the invention, the reactive groups of the precursor can be subjected to an addition reaction in a very controlled manner. By selecting suitable values for reaction parameters such as time, temperature and addition reaction catalyst concentration, the reactive groups of the precursor can be modified to any desired level, even essentially completely.

Another advantage of the present invention is that the product resulting from the addition reaction (wherein the cyclic monomers are modified through the addition of carrier compounds with refractive index modifying groups) can be purified more easily than viscous polysiloxanes of the prior art. Cyclic precursors that have reacted sufficiently may have opened-up and may partly have polymerized rendering these modified precursors insoluble, while cyclic precursors that have not reacted are still soluble in solvents such as methanol and can easily be removed.

Also, after purification, the amount of unreacted reactive groups, such as vinyl groups, present in a polysiloxane of the present invention is constant. When preparing a polysiloxane of the present invention under optimal conditions for the addition reaction, a very small number of unreacted reactive groups usually remains. Under these circumstances, co-polymerization may for example require the incorporation of extra vinylsiloxane units to reach the amount of free vinyl groups advantageous for proper cross-linking at high molecular weights of the polymer (usually about 1 mol%).

Conversely, polysiloxanes with refractive index modifying groups prepared by methods of the prior art usually possess free unreacted reactive groups in uneven distribution or to an unknown extent, which hampers effective control of the cross-linking reaction and of the addition reaction.

A method according to the invention, however, wherein addition reactions to the monomeric precursor are performed, results in an even distribution of free unreacted reactive groups. Further, the reaction may take place at relatively low temperatures, whereby the incidence of undesired cross-linking between reactive groups of the individual precursors is reduced.

Another advantage of the invention is that under optimal conditions, ring opening of the cyclic precursor may take place spontaneously during the addition (or modification) reaction. Generally, ring opening of cyclic siloxanes with large substituents is perceived as difficult due to steric hindrance. Spontaneous ring opening during a cyclic siloxane modification reaction according to the invention occurs particularly well when large substituents such as triphenyl silane are used and may in such instances take place without the aid of (co)polymerization catalysts at temperatures in excess of only about 50°C.

Although spontaneous ring opening is advantageous to certain embodiments of the present invention, (co-)polymerization catalysts may be used when a process for the preparation of a polysiloxane according to the invention is performed by an addition reaction with certain combinations of cyclic monomeric precursors and clustered configurations of refractive index modifying groups. In particular, in those instances that the addition reaction will not result in spontaneous ring opening and subsequent polymerization, (co-)polymerization catalysts may be added or reaction temperatures may be increased. The person skilled in the art will readily understand how the reaction conditions can be optimized in the combined addition reaction and (co-)polymerization reaction for various combinations of cyclic precursor monomers and clustered configurations of refractive index modifying groups.

A reaction mixture for performing an addition reaction according to the present invention preferably comprises a cyclic siloxane and clustered configuration of refractive index modifying groups in a molar ratio of about 10: 1 to about 1:10, more preferably of about 1:1 to about 1:10, even more preferably of about 1:4. A reaction mixture according to the invention further preferably comprises an addition reaction catalyst in an amount depending on the type of catalyst, but in the case of platinum divinyl tetramethyl disiloxane an amount of about 10-200 ppm, preferably of about 50 ppm can be used. Optionally, a reaction mixture may comprise a (co-)polymerization catalyst and additives such as UV-blockers. It is advantageous to exclude air from the reaction mixture. The reaction preferably takes place under an inert atmosphere, such as a nitrogen atmosphere.

By carefully selecting the reaction conditions for the addition reaction to the precursor and the reactants, massive ring-opening will occur and polymerization will take place. Suitable reaction conditions include a reaction temperature of about 30- 150 °C, preferably about 70 - 100 °C and most preferably about 90 °C. The mixture is preferably stirred at the reaction temperature and the reaction is allowed to take place for a period of about 6 - 168 hours, preferably 72 - 120 hours. Optionally, the reaction can be stopped at 75-99 % of the maximum level of additions and polymerisation will still occur.

One suitable method for the preparation of a high refractive index polysiloxane homo-polymer of the invention comprises the modification of the cyclic siloxane precursor tetravinyl tetramethyl cyclotetra siloxane with triphenyl silane, which is exemplified in example 1. A schematic representation of the modification of trivinyl pentamethyl tetrasiloxane with triphenyl silane is illustrated in figure 3.

As an addition reaction catalyst, any catalyst for hydrosilylation such as platinum group metal components or Speiers catalyst can be used. In a preferred embodiment Karstedt catalyst (platinum divinyl tetramethyl disiloxane) is used.

An alternative reaction route for the preparation of a high refractive index polysiloxane homo-polymer of the invention is formed by producing the clustered configuration of refractive index modifying groups as a Grignard-reagent and contacting this reagent with a cyclic hydride-containing siloxane precursor. Such a method is well known in the art.

A high refractive index polysiloxane co-polymer may be prepared according to a method of the invention by performing the addition reaction to a cyclic siloxane monomer in the presence of other cyclic siloxane monomers (termed co-monomers hereinafter) or with other (low refractive index) polysiloxanes to obtain co-polymers of a specific refractive index or with increased flexibility, increased mechanical strength or with a variety of functional groups (e.g. cross-linking enhancing groups such as vinyl or hydride groups in Pt-catalyzed systems or alkoxy groups).

Thus, high refractive index polysiloxane co-polymers can be derived from high refractive index polysiloxane homo-polymers according to the invention in one of several ways.

For example, a high refractive index polysiloxane homo-polymer can be mixed (in polymeric form) with other polysiloxanes, in the presence of a (co-)polymerization catalyst (and optionally a solvent), and allowed to copolymerize thereby forming a high refractive index polysiloxane co-polymer. Also, a high refractive index polysiloxane can be prepared by performing an addition reaction to a cyclic siloxane according to the invention in the presence of a polysiloxane.

Alternatively, in order to prepare a high refractive index polysiloxane co-polymer, other siloxane monomers, or co-monomers, may be administered to the reaction mixture for preparing a high refractive index polysiloxane homo-polymer of the invention as set out above, as such administration will result in co-polymerization between the various monomers and the formation of a high refractive index polysiloxane co-polymer.

A large number of cyclic co-monomers are suitable for use in the above described co-polymerization for the preparation of a high refractive index polysiloxane co-polymer according to the invention. Co-monomers may or may not comprise refractive index modifying groups themselves. They may or may not comprise reactive groups, such as hydride or vinyl groups. Examples of cyclic co-monomers that can be co-polymerized in a combined addition/polymerization reaction of cyclic siloxanes according to the invention are tetravinyl tetramethyl cyclo tetrasiloxane, octamethyl cyclo tetrasiloxane, hexamethyl cyclo trisiloxane, trimethyl triphenyl cyclo trisiloxane, tetramethyl tetraphenyl cyclo tetrasiloxane, trifluoro propylmethyl cyclo trisiloxane, hexaphenyl cyclo trisiloxane, and octaphenyl cyclo tetrasiloxane.

It may be clear from the above that various cyclic siloxanes may be used in combination as co-monomers or as monomeric precursors in methods for preparing a high refractive index polysiloxane homo-polymer or a high refractive index polysiloxane co-polymer according to the invention.

One method for preparing a co-polymer according to the invention may comprise the purification of a triphenyl modified siloxane polymer (a high refractive index polysiloxane homo-polymer) by stirring it in boiling methanol and recovering it by precipitation and decanting the boiling methanol. The resulting white paste can be dried in a vacuum oven until a transparent material is obtained. At room temperature such material may appear as a glassy solid. This material can then be mixed with octamethyl cyclo tetrasiloxane and a small amount of tetravinyl tetramethyl cyclo tetrasiloxane at the reaction temperature of between 10 - 200 °C, but preferably 100 - 150 °C under inert atmosphere (nitrogen). Then, a (co-)polymerization catalyst such as potassium silanolate dissolved in purified, dried dimethyl sulfoxide (DMSO) can be added, preferably 10⁻⁶ to 10⁻⁵ mole of potassium silanolate per gram of mixture is used. Reaction then may preferably take place under an inert atmosphere for a duration of between 1 - 200 hours, until a transparent viscous material is formed. Preferably the reaction time is 96 hours or more to obtain complete randomization of siloxane groups in the co-polymer. Instead of potassium silanolate, other basic catalysts may be used as (co-)polymerization catalyst, such as potassium hydroxide or sodium hydroxide. The presence of DMSO is not essential but increases the reaction rate for potassium salts.

Co-polymers with a refractive index varying from 1.4 to about 1.6 can be produced by co-polymerization of highly phenylated siloxane polymers and siloxane polymers with a lower degree of aromatic substitution or even phenyl-free siloxane polymers using a method described hereinabove.

Co-polymers according to the invention have the formula: 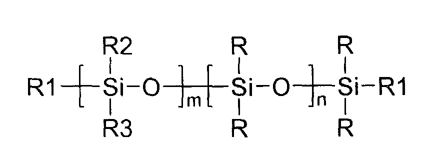
Wherein R is independently selected from alkyl radicals, substituted alkyl radicals, aryl radicals, substituted aryl radicals, aromatic radicals such as naphtyl radicals, cycloalkyl radicals, arylalkyl radicals, allyl radicals, vinyl and hydride;
R1 is independently selected from alkyl radicals, substituted alkyl radicals, aryl radicals, substituted aryl radicals, aromatic radicals such as naphtyl radicals, cycloalkyl radicals, arylalkyl radicals, allyl radicals, vinyl and hydride, monovalent hydrocarbon radicals having a multiple bond or substituted equivalents thereof, monovalent hydrocarbon radicals having a siliconhydride group, and triphenyl siloxane groups, but preferably comprises a vinyl or hydride group;
R2 is preferably a triphenyl silylalkyl radical, but can be selected from the group of triarylsilyl alkyl radicals, diarylalkyl silylalkyl radicals, aryl dialkylsilyl alkyl radicals, trialkyl alkylsilyl radicals, tricycloalkyl silylalkyl radicals, dicycloalkyl alkylsilyl alkyl radicals, cycloalkyl dialkyl silylalkyl radicals;
R3 is independently selected from alkyl radicals, substituted alkyl radicals, aryl radicals, substituted aryl radicals, aromatic radicals such as naphtyl radicals, cycloalkyl radicals, arylalkyl radicals, allyl radicals, vinyl and hydride, but is preferably an alkyl radical, more preferably a methyl radical;
m is between 1 and about 2,000,000, preferably between about 100,000 and about 1,000,000;
n is between 0 and about 2,000,000, preferably between about 500,000 and about 1,000,000.

As end blocking groups for example divinyl tetramethyl disiloxane can be used.

The polymeric resin may further contain endgroups (empirical formula R₃SiO_{0.5}), and branching points and T-resins (empirical formula RSiO_{1.5}) and tetrafunctional units (empirical formula SiO₂), structures known to those skilled in the art, to improve material properties for the application involved. These organopolysiloxane copolymers are known in the art as MQ resins.

The presence of unreacted vinyl groups as part of siloxane co-monomers or co-polymers, allows cross-linking of the co-polymer as described earlier. Unreacted vinyl groups may also deliberately be introduced by choosing reaction conditions in such a way that not all reactive groups (such as hydride or vinyl groups) on the precursor monomers have reacted.

As cross-linkers, any siloxane co-polymer with hydride groups or with vinyl groups can be used in a method of the present invention, depending on the composition of the polymer that is to be cross-linked. For example, copolymers of methyl hydrosiloxane and phenyl methylsiloxane, co-polymers of methyl hydrosiloxane and diphenylsiloxane, and co-polymers of methyl vinylsiloxane and phenyl methylsiloxane and/or combinations of such compounds can be used. Also tetrakis (dimethylsiloxy) silane, dimethylsilane, methylsilane and/or 1,1,3,3-tetramethyl disiloxane can be used as cross-linking reagent. Alternative cross-linking systems can also be used.

Functional groups that contribute to cross-linking can be introduced in the co-polymer. Examples are alkoxy, acetoxy, ketoxime, amine, (meth)acrylate en epoxy functional siloxane copolymers. (Meth)acrylate containing units such as acryloxypropyl can undergo UV - and visible light cross-linking when suitable photo-initiators are present. Epoxy-modified siloxane units e.g. can undergo electron beam curing.

Cross-linking preferably should not proceed too rapidly at room temperature. In order to improve handling and increase processing times, for example required during mixing of different co-polymer components and injection molding, cross-linking inhibitors such as 1,2,3,4-tetramethyl-1,2,3,4-tetravinyl-cyclotetrasiloxane may be used. Such inhibitors may be added to the reaction mixture of the addition reaction in amounts of 0.01 - 10 wt. % based on the weight of the polymer.

Prior or during polymerization or co-polymerization or cross-linking, additives such as UV-blocking agents or other additives such as dyes may be administered to the reaction mixtures. As UV blocking agents, vinyl functional or hydride functional benzotriazol, vinyl functional or hydride functional 2-hydroxy benzophenones, allyl benzophenones (monoallyl benzophenone) and vinyl functional or hydride functional benzotriazoles may be used in amounts of 0.01 - 5 wt. %based on the weight of the polymer.

A high refractive index polysiloxane homo-polymer according to an embodiment of the present invention preferably exhibits a refractive index of at least 1.6, more preferably at least 1.65. In accordance with the invention the refractive index is measured by Abbé-type refractometry.

High refractive index polysiloxane homo-polymers as well as a high refractive index polysiloxane co-polymers according to the present invention are very suitable for use in a multitude of applications. Especially in those applications where light refraction or optical appearance is important, such as in optical lenses, optical fibers, intra-ocular devices such as intra-ocular lenses for cataract, refractive and reconstruction surgery as well as for occluders and tear duct devices, in contact lenses, ophthalmic applications, glasses, windows, windshields, transparent coatings and in cosmetic products like hair care products the material of the present invention can be beneficially applied.

Formation of intra-ocular lenses or optics from the high refractive index polysiloxane co-polymers of the present invention may be accomplished by liquid injection molding or by cast or compression molding or other types of molding. These processes are well known to the art. Suitable vulcanization temperatures for such processes are between 20- 200 °C.

The following non-limiting examples illustrate several embodiments of the present invention.

### Example 1.

### Modification of tetravinyl tetramethyl cyclo tetrasiloxane with triphenyl silane.

Tetravinyl tetramethyl cylco tetrasiloxane and triphenyl silane were mixed in a molar ratio of 1 to 4. As an addition reaction catalyst, an amount of 50 ppm of platinum divinyl tetramethyl disiloxane was added as a 2.5 wt.% solution in xylene. Air was excluded by replacing it with a nitrogen atmosphere and the mixture was heated to 90 °C. The mixture was stirred at the reaction temperature for 120 hours.

After the reaction time the mixture was cooled down and 2 volume parts of methanol were added. After stirring for 4 hours in boiling methanol, stirring was stopped and the product was allowed to precipitate for 2 hours. Boiling methanol was decanted. An amount of 2 volumes of fresh methanol was added and the product was stirred in boiling methanol overnight. Again stirring was stopped, the polymer allowed to precipitate and boiling methanol was decanted. The material was dried overnight in a vacuum oven at 70 °C and then cooled.

A transparent glassy solid was obtained which by exhibited a refractive index of 1.65 by using Abbé-type refractometry. Co-polymerization experiments showed that the refractive index of the high refractive index polysiloxane homo-polymer was about 1.66.

NMR analysis showed that unreacted triphenyl silane was removed and that vinyl groups had almost completely disappeared. Instead, protons from bridging alkyl groups, resulting from the addition reaction, as well as protons from phenyl groups were present.

### Example 2

### Preparing a co-polymer

An amount of 1 g of the high refractive index polysiloxane homo-polymer of example 1 was mixed with 0.55 g of octamethyl cyclo tetrasiloxane and 0.5 mg of tetravinyl tetramethyl cylo tetrasiloxane. To this mixture an amount of 1 × 10⁻⁵ mol of potassium trimethyl silanolate per g of mixture was added as a (co-)polymerization catalyst. The catalyst was added as a 1 M solution in DMSO. The mixture was stirred at 140 °C for 120 hours and a transparant polymer was obtained. After cooling to 70 °C and adding toluene as a solvent the catalyst was neutralized and chloro dimethyl vinylsilane was added as an endcapper. Residual monomers were evaporated from the reaction mixture along with the toluene.

### Example 3

### Manufacturing of an intra-ocular lens (IOL):

The co-polymer of example 2 was divided into two aliquots. To one part, hydride functional cross-linker was added in a ratio of 1:1 hydride groups to vinyl groups in the material after mixing of the two parts. To the other part 10 ppm of platinum divinyl tetramethyl disiloxane was added. An amount of 0.1 wt.% of 1,2,3,4-tetramethyl-1,2,3,4-tetravinyl-cyclotetrasiloxane inhibitor, based on the weight of the polymer, was added to the part with the hydride cross-linker, and a UV-blocker (0.1 wt.%, based on the weight of the polymer) was added to both parts.

The two parts were mixed in an extruder and formation of IOL bodies was accomplished by liquid injection molding into a mould at a vulcanization temperature of 110 °C. Post-curing was performed in an oven at a temperature of 120 °C for a periods of 2 hours.

## Claims

1. High refractive index polysiloxane homo-polymer, comprising a clustered configuration of refractive index modifying groups chemically bonded to the polysiloxane backbone via an alkanediyl-bridge such that one alkanediyl-bridge binds at least two refractive index modifying groups to said backbone.

2. Polysiloxane homo-polymer according to claim 1, wherein the refractive index modifying groups are independently selected from the group consisting of cyclic, straight chain and branched chain hydrocarbon moieties.

3. Polysiloxane homo-polymer according to claim 1, wherein the refractive index modifying groups are independently selected from the group consisting of aromatic groups, aryl groups, arylalkyl groups, cycloalkyl groups and branched alkyl groups.

4. Polysiloxane homo-polymer according to claim 3, wherein the refractive index modifying groups are phenyl groups.

5. Polysiloxane homo-polymer according to any of the preceding claims, wherein the alkanediyl-bridge is an ethanediyl bridge.

6. Polysiloxane homo-polymer according to any of the preceding claims, wherein the clustered configuration of refractive index modifying groups comprises a central silicon atom.

7. Polysiloxane co-polymer comprising a polysiloxane homo-polymer according to any of the claims 1-6.

8. Co-polymer according to claim 7, wherein said polysiloxane homo-polymer is co-polymerized with a polymer derived from a cyclic co-monomer selected from the group consisting of tetravinyl tetramethyl cyclo tetrasiloxane, octamethyl cyclo tetrasiloxane, hexamethyl cyclo trisiloxane, trimethyl triphenyl cyclo trisiloxane, tetramethyl tetraphenyl cyclo tetrasiloxane, trifluoropropyl methyl cyclo trisiloxane, hexaphenyl cyclo trisiloxane and octaphenyl cyclo tetrasiloxane.

9. Co-polymer according to Formula I, 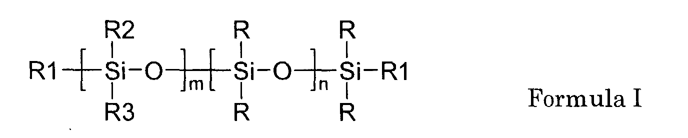 wherein
R is independently selected from the group consisting of alkyl radicals, substituted alkyl radicals, aryl radicals, substituted aryl radicals, aromatic radicals, cycloalkyl radicals, arylalkyl radicals, allyl radicals, vinyl and hydride;
R1 is independently selected from the group consisting of alkyl radicals, substituted alkyl radicals, aryl radicals, substituted aryl radicals, aromatic radicals, cycloalkyl radicals, arylalkyl radicals, allyl radicals, vinyl, hydride, optionally substituted monovalent hydrocarbon radicals having a multiple bond, optionally substituted monovalent hydrocarbon radicals having a siliconhydride group, and triphenylsiloxane groups;
R2 is selected from the group consisting of triaryl silylalkyl radicals, diarylalkyl silylalkyl radicals, aryldialkyl silylalkyl radicals, trialkyl alkylsilyl radicals, tricycloalkyl silylalkyl radicals, dicycloalkyl alkyl silylalkyl radicals, cycloalkyl dialkyl silylalkyl radicals;
R3 is independently selected from the group consisting of alkyl radicals, substituted alkyl radicals, aryl radicals, substituted aryl radicals, aromatic radicals, cycloalkyl radicals, arylalkyl radicals, allyl radicals, vinyl and hydride;
m is between 1 and about 2,000,000;
n is between 0 and about 1,000,000.

10. Co-polymer according to claim 9, wherein R2 is a triphenyl silylethyl radical.

11. Co-polymer according to claim 9 or 10, wherein R1 is vinyl or hydride; and R3 is a methyl radical.

12. Polysiloxane homo-polymer or polysiloxane co-polymer according to any of the claims 1-11, further comprising an additive, such as a UV-blocking agent, a dye or pigment and/or a cross-linking inhibitor.

13. Use of a polysiloxane homo-polymer or polysiloxane co-polymer according to any of the claims 1-12 as a material for the manufacturing of an intra-ocular lens.

14. Method for preparing a high refractive index polysiloxane homo-polymer comprising the steps of
a) chemically modifying a cyclic siloxane by performing an addition reaction in the presence of an addition reaction catalyst which reaction links a clustered configuration of refractive index modifying groups to the siloxane backbone of said cyclic siloxane via an alkanediyl-bridge such that at least two of said refractive index modifying groups are bound to said backbone of said cyclic siloxane via a single alkanediyl-bridge;
b) allowing ring-opening of the cyclic siloxane; and
c) facilitating subsequent polymerization of the resulting reaction products thereby obtaining the high refractive index polysiloxane homo-polymer.

15. Method for preparing a high refractive index polysiloxane homo-polymer according to claim 14, comprising chemically modifying a cyclic siloxane of Formula II 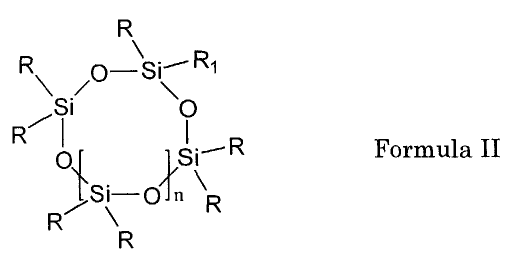 wherein
R is independently alkyl, vinyl or hydride;
R₁ is vinyl;
and n = 0, 1, 2 or 3;
by performing the steps of
a) reacting said cyclic siloxane with triphenylsilane, diphenylsilane, triphenyl dimethyl disiloxane, or diphenylalkyl dimethyl disiloxane in the presence of an addition reaction catalyst;
b) allowing ring-opening of the cyclic siloxane; and
c) facilitating subsequent polymerization of the resulting reaction products thereby obtaining the high refractive index polysiloxane homo-polymer.

16. Method for preparing a high refractive index polysiloxane homo-polymer according to claim 14, comprising chemically modifying a cyclic siloxane of Formula II 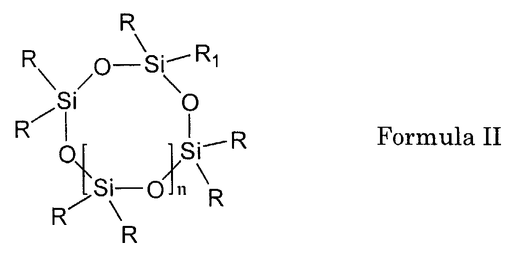 wherein
R is independently alkyl, vinyl or hydride;
R₁ is hydride;
and n = 0, 1, 2 or 3;
by performing the steps of
a) reacting said cyclic siloxane with triphenyl vinylsilane, diphenylalkyl vinylsilane, phenyl dialkyl vinylsilane, triphenyl dimethyl vinyldisiloxane, diphenylethene, 3,3,3-triphenyl-1-propene, 3,3-diphenyl-1-propene or any other hydride reactive polyphenyl, in the presence of an addition reaction catalyst;
b) allowing ringopening of the cyclic siloxane; and
c) facilitating subsequent polymerization of the resulting reaction products thereby obtaining the high refractive index polysiloxane homo-polymer.

17. Method according to any of the claims 14 -16, wherein said cyclic siloxane is a cyclic tetrasiloxane.

18. Method according to claims 17, wherein said cyclic tetrasiloxane is reacted with a triphenylsilane or triphenyl vinylsilane.

19. Method for preparing a high refractive index polysiloxane co-polymer, wherein an addition reaction for chemically modifying a cyclic siloxane according to claim 14 is performing in the presence of a cyclic siloxane co-monomer or a polysiloxane.

20. Method for preparing a high refractive index polysiloxane co-polymer, wherein a high refractive index polysiloxane homo-polymer obtained by a method according to any of the claims 14-18 is co-polymerized with a cyclic siloxane co-monomer or with a polysiloxane.

21. Method according to claim 19 or 20, wherein the cyclic siloxane co-monomer is selected from the group consisting of tetravinyl tetramethyl cyclo tetrasiloxane, octamethyl cyclo tetrasiloxane, hexamethyl cyclo trisiloxane, trimethyl triphenyl cyclo trisiloxane, tetramethyl tetraphenyl cyclo tetrasiloxane, trifluoropropyl methyl cyclo trisiloxane, hexaphenyl cyclo trisiloxane and octaphenyl cyclo tetrasiloxane.
